# EUROPEAN PATENT SPECIFICATION

(11) **EP 4 436 654 B1**
(45) Date of publication and mention of the grant of the patent: **29.10.2025**
(21) Application number: 22925236.6
(22) Date of filing: 23.09.2022
(51) Int. Cl.: A61N 1/36, A61B 18/12, A61N 1/32

(54) **ENHANCED FOCAL STIMULATION BY SPATIOTEMPORAL SUMMATION OF NANOSECOND ELECTRIC PULSES**
VERBESSERTE FOKALE STIMULATION DURCH RÄUMLICH-ZEITLICHE SUMMIERUNG VON NANOSEKUNDEN ELEKTRISCHEN IMPULSEN
STIMULATION FOCALE AMÉLIORÉE PAR SOMMATION SPATIO-TEMPORELLE D'IMPULSIONS ÉLECTRIQUES DE NANOSECONDE

(30) Priority: 26.11.2021 US 202163283371 P
(43) Date of publication of application: 02.10.2024
(73) Proprietor: Old Dominion University Research Foundation, Norfolk, VA 23508 (US)
(72) Inventor: PAKHOMOV, Andrei G., Norfolk, Virginia 23508 (US); KIM, Vitalii, Norfolk, Virginia 23508 (US); SEMENOV, Iurii, Norfolk, Virginia 23508 (US); GUDVANGEN, Emily, Norfolk, Virginia 23508 (US)
(74) Representative: IP Sextant s.r.l.
(86) International application number: PCT/US2022/076938
(87) International publication number: WO 2023/167747

(56) References cited:
- WO-A2-2011/047387
- US-A1- 2021 268 274
- US-B2- 8 926 606

## Description

### BACKGROUND

Pulsed electric field treatments can be used to deliver energy to provide therapeutically beneficial bioeffects to cells and/or tissue such as stimulation, electroporation, ablation, and gene electro-transfer (hereinafter collectively referred to as "stimulation" or "electrostimulation"), or any other modification. Stimulation can be used to manipulate biological function in various applications, which may be referred to generically as "modifying" the target tissue or cell(s). WO2011/047387 describes systems, methods and devices for electroporation-based therapies (EBTs).

In many cases, it is desirable that the stimulation bioeffects from pulsed electric field treatments are evoked in a targeted local area, but not in other non-targeted areas. This targeted approach may be referred as "focal stimulation."

### SUMMARY OF THE DISCLOSURE

The invention is defined by the independent claim 1. The non-claimed methods and apparatuses (e.g., systems and devices) described herein are useful in any applications benefitting from the focal application of energy to a cell or tissue from one electrode with minimized effect at other electrodes. These methods and apparatuses enable controlled depth and direction of the pulsed electric field treatments by utilizing spatiotemporal summation of trains of unipolar sub-microsecond electric pulses (including nanosecond pulses) from a set of electrodes in triangular or pyramid configurations. For example, one or more pairs of emitting electrodes are arranged to each concurrently emit a train of sub-microsecond unipolar pulses that are 180 degrees out of phase so that the pulses cancel around the emitting electrodes but sum specifically at a dedicated ground electrode that is positioned apart from the one or more pairs of emitting electrodes. In any of these methods and apparatuses, the sub-microsecond unipolar pulses may sum at the target tissue without substantially modifying non-target tissue or cell, including the non-target tissue/cells adjacent to and/or between the one or more pairs of emitting electrodes.

For example, described herein are methods of applying energy to a target tissue or cell. These methods may be methods of applying, steering, directing, and/or focusing energy to a target cell, group of cells or tissue either within a body or outside of the body (e.g., in culture). For example, described herein are methods including: positioning a ground (e.g., targeting) electrode on or adjacent to the target tissue or cell; applying a first train of sub-microsecond unipolar pulses from a first electrode and a second train of sub-microsecond unipolar pulses from a second electrode, wherein the first and second trains of sub-microsecond unipolar pulses are concurrently applied and are out of phase (such as 180 degrees out of phase) so that the first and second trains of sub-microsecond unipolar pulses sum at the target tissue or cell.

A method of applying energy to a target tissue or cell may include: positioning a ground or targeting electrode relative to a target tissue or cell so that the target tissue or cell is closer to the ground or targeting electrode than to a first or a second electrode; applying a first train of sub-microsecond unipolar pulses from the first electrode and a second train of sub-microsecond unipolar pulses from the second electrode, wherein the first and the second trains of sub-microsecond unipolar pulses are concurrently applied, are 180 degrees out of phase, and are each applied for a burst duration, so that the first and second trains of sub-microsecond unipolar pulses sum at the target tissue or cell to form a unipolar pulse having a pulse width that is equivalent to the burst duration without substantially modifying non-target tissue or cell.

The ground or targeting electrode may be positioned independently of the first and second electrodes or it may be positioned as a group or unit with the first and second (and in some examples, additional pairs) electrodes. Any of the methods and apparatuses described herein may include positioning the first electrode and the second electrode and the targeting electrode so that the target tissue or cell is between a line formed between the first and second electrodes and the targeting electrode. In any of these methods and apparatuses, positioning the targeting electrode may include inserting a needle electrode (comprising the targeting electrode) into the target tissue.

Positioning a targeting electrode on or adjacent to, or in proximity to, the target tissue or cell may include positioning the targeting electrode relative to the target tissue or cell(s) (or vice versa). For example, the targeting electrode may be applied or inserted into/on a target tissue region so that it is sufficiently close to the target tissue or cell(s) or in some implementations, is at a distance from the target tissue (e.g., up to 10 cm). In general, the targeting electrode may be positioned so that the target tissue or cell(s) is within the comet-shaped region around the targeting electrode, the tail of which extends towards the electrodes applying the concurrent unipolar pulse trains. Summation of the concurrent trains of unipolar pulses may result in modification of the tissue or cell(s) within this comet-shaped region. For example, when the target tissue is a brain cortex and non-invasive treatment rather than a surgery is desirable, the targeting electrode may be placed on the skin above the brain cortex so that electric treatment will spread from the targeting electrode deeper into the cortex. Therefore, as used herein, adjacent to the target tissue or cell(s) may include in contact with the target tissue or cell(s) or within a proximity (e.g., reasonably close proximity) without necessarily being in contact with (e.g., within several cm of the targeting electrode, within about 1 cm of the targeting electrode, within about 0.6 mm of the targeting electrode, within about 0.5 mm of the targeting electrode, within about 0.4 mm of the targeting electrode, within about 0.35 mm of the targeting electrode, within about 0.3 mm of the targeting electrode, etc.). A penetration depth of the electric treatment, and therefore a corresponding distance from the targeting electrode to the target tissue or cell, is proportional to the size of the electrodes. For example, some of the methods may include positioning the targeting electrode on or adjacent to the target tissue within about 0.3-0.5 mm of the targeting electrode. However, a larger electrode surface will allow a broader and deeper penetration, and therefore, "adjacent to the target tissue" may include a longer distance in centimeters between the targeting electrode and the target tissue or cell.

The first and second trains may be concurrently applied so the first electrode operates as a ground electrode when the second electrode is delivering a unipolar pulse of the second train of sub-microsecond unipolar pulses, and the second electrode operates as a ground electrode when the first electrode is delivering a unipolar pulse of the first train of sub-microsecond unipolar pulses.

The pulses may be any appropriate shape, such as rectangular (e.g., square waves) or approximately rectangular, sinusoidal, triangular, etc. For example, applying the first train of sub-microsecond unipolar pulses and the second train of sub-microsecond unipolar pulses may include applying a first train of rectangular sub-microsecond unipolar pulses and a second train of rectangular sub-microsecond unipolar pulses.

The intensity (e.g., amplitude) of the pulses may be adjusted (increased and/or decreased). In some examples the amplitude may be adjusted to adjust the depth of penetration of the applied energy. In some examples the amplitude may be adjusted dynamically (including automatically or semiautomatically) by the apparatus, to prevent or reduce modifying non-target tissue or cells. In some examples the amplitude may be adjusted manually. In some examples the amplitude may be adjusted to increase the depth or size of the region being treated (e.g., increasing the amplitude). For example, any of these methods may include adjusting an amplitude of the sub-microsecond unipolar pulses of the first train of sub-microsecond unipolar pulses and of the second train of sub-microsecond unipolar pulses to modify an amplitude of a summed unipolar pulse at the target tissue or cell.

In any of these methods and apparatuses an individual pulse width (typically, an elapsed time between the leading and trailing edges of a single pulse of energy) of the unipolar pulses in the first and second trains of sub-microsecond pulses may be within the sub-microsecond range (e.g., having a pulse duration of 10 microseconds or less, for example, less than 999 ns). The pulse width may be 900 ns or less, 800 ns or less, 700 ns or less, 600 ns or less, 500 ns or less, 400 ns or less, 300 ns or less, 200 ns or less, 100 ns or less, etc. The pulse width may be adjustable. The pulse width of the pulses in the first and second trains may be the same.

As used herein the phrase "target tissue" may refer to an isolated (e.g., external to a human or animal subject) or *in vivo* tissue and may refer to a region or sub-region of a tissue. The target tissue may include a cell or cells (or a type or class of cell or cells) within a broader tissue. The phrase "target cell" may include one cell or multiple cells. Unless the context specifies otherwise, the phrase target tissue may be used equivalently and interchangeably with target cell or target cells. Similarly, non-target tissue may be used equivalently and interchangeably with non-target cells.

In general, these methods and apparatuses may be used to modify the target tissue (e.g., tissue region) or cell (e.g., group of cells). Thus, applying the first train of sub-microsecond unipolar pulses from the first electrode and the second train of sub-microsecond unipolar pulses from the second electrode may further comprise modifying the target tissue or cell. For example, modifying may include electroporating the target tissue or cell, exciting the target tissue or cell, and/or ablating the target tissue or cell.

In some examples the applicator may be configured having a triangular configuration, in which the first and second electrode form the base of the triangle, and the ground/targeting electrode (positioned near the tissue) forms the tip of the triangle. Alternatively, the same ground electrode may be used with other pairs of electrodes, e.g., forming pyramidal applicators, etc. Thus, any of the methods and apparatuses described herein may include using additional pairs of electrodes to concurrently apply trains of sub-microsecond pulses that are out of phase, such as 190 degrees out of phase, 170 degrees out of phase, 180 degrees out of phase. For example, any of these methods may be configured so that the first train of sub-microsecond unipolar pulses is applied from both the first electrode and a third electrode and the second train of sub-microsecond unipolar pulses is applied from both the second electrode and a fourth electrode.

In any of these methods and apparatuses the first and second trains of sub-microsecond unipolar pulses are applied to sum at the target tissue or cell to form a unipolar pulse having a pulse width that is equivalent to a burst duration of the first train and the second train. Thus, any of these methods and apparatuses may adjust the burst duration of the first and second trains to increase or decrease the pulse width of the summed unipolar pulse at or near the ground/targeting electrode. The burst duration of the first and the second train of sub-microsecond unipolar pulses may generally be approximately the same (plus or minus one or two pulses).

In any of these methods and apparatuses, the burst duration of the first and second trains of sub-microsecond unipolar pulses may be adjustable. For example, the first train of sub-microsecond unipolar pulses and the second train of sub-microsecond unipolar pulses may each have a burst duration of between 1 microsecond and 100 millisecond so that the first and the second trains of sub-microsecond unipolar pulses sum at the target tissue or cell to form a unipolar pulse having a pulse width of between 1 microsecond and 100 millisecond.

The methods and apparatuses described herein may also include adjusting the electrical field strength of the first and second trains of sub-microsecond unipolar pulses. In any of these methods and apparatuses, the electric field strengths of the first and second trains of sub-microsecond unipolar pulses may be 20 kV/cm or less (e.g., 15 kV/cm or less, 10 kV/cm or less, 8 kV/cm or less, 7 kV/cm or less, 6 kV/cm or less, 5 kV/cm or less, 4 kV/cm or less, 3 kV/cm or less, 2 kV/cm or less, 1 kV/cm or less, etc.).

Also described herein are apparatuses (e.g., systems, devices, etc., including software, firmware and/or hardware) for performing any of these methods. In particular, described herein are systems for applying energy to a target tissue (e.g., sub-region of tissue) or cell(s). These systems may be configured specifically to selectively target the target tissue or cell using these methods.

For example, described herein are systems for applying energy to a target tissue or cell that include: a first output configured to couple to a first one or more electrode(s); a second output configured to couple to a second one or more electrode(s); a third output configured to couple to a targeting electrode; a pulse generator configured to generate a first train of sub-microsecond unipolar pulses and a second train of sub-microsecond unipolar pulses, wherein the first and the second trains of sub-microsecond unipolar pulses are out of phase (for example, 180 degrees out of phase); and a controller configured to apply the first train of sub-microsecond pulses to the first output concurrently with the second train of sub-microsecond pulses to the second output, and to couple the third output to the targeting electrode.

In some examples a system for applying energy to a target tissue or cell includes: a pulse generator configured to generate a first train of sub-microsecond unipolar pulses and a second train of sub-microsecond unipolar pulses, wherein the first and second trains of sub-microsecond unipolar pulses are 180 degrees out of phase; a first electrode coupled to the pulse generator through a first output; a second electrode coupled to the pulse generator through a second output; a targeting electrode coupled to the pulse generator through a third output; and a controller configured to apply the first train of sub-microsecond unipolar pulses to the first output concurrently with the second train of sub-microsecond unipolar pulses to the second output, and to couple the third output to the targeting electrode. The first electrode may comprise one or more first electrodes and the second electrode may comprise one or more second electrodes.

Thus, any of these systems may optionally include the first electrode (which may be coupled to the pulse generator through the first output), the second electrode (which may be coupled to the pulse generator through the second output), and the ground (targeting) electrode (which may be coupled to the pulse generator through the third output). Optionally, the electrodes may be part of one or more applicators. For example, an applicator may include the first, second and targeting electrodes (and optionally, additionally one or more additional pairs of electrodes); in some examples separate applicators may be used with the targeting and first and second electrodes (or each electrode may include a separate applicator). For example, the first electrode and the second electrode may be part of an applicator.

The first, second and targeting electrodes may be any appropriate type of electrode, including non-penetrating (e.g., surface electrode, flat electrode, plate electrode, disk electrode, etc.), or penetrating electrodes (e.g., needle electrode, knife electrode, etc.). For example, the targeting electrode may comprise a needle electrode. The targeting electrode and the first and the second (or other electrode pairs) may be the same type of electrode or different types of electrodes.

In any of these systems the controller may include hardware (e.g., circuitry, memory, input(s), output(s), etc.). The controller may be integrated with the pulse generator or separate from (but connected to) the pulse generator. The controller may comprise or be operatively connected to one or more processors. The controller may include one or more user inputs for controlling operation of the apparatus. Inputs may include keyboards, touchscreens, knobs, dials, sliders, etc. For example, the controller may be configured to adjust an amplitude (e.g., V of kV) or an electric field strength (e.g., kV/cm) of the first train of sub-microsecond pulses and the second train or sub-microsecond pulses. In some examples, the controller may be configured to adjust an electric field strength of the first train of sub-microsecond pulses and the second train of sub-microsecond pulses such that the electric field strength of a summed unipolar pulse at the targeting electrode is between 0.01 kV/cm and 10 kV/cm. The controller may be configured to allow manual adjustment of the amplitude or the electric field strength, or in some examples, configured to automatically adjust the amplitude or the electric field strength.

In any of these apparatuses, the controller may be configured to adjust a burst duration of the first train of sub-microsecond pulses and the second train or sub-microsecond pulses. For example, the controller may be configured to adjust a burst duration of the first train of sub-microsecond pulses and the second train of sub-microsecond pulses so that a pulse width of a summed unipolar pulse at the ground electrode is between 1 microsecond and 100 milliseconds. As mentioned, the controller may be configured to allow manual operation of the burst duration or in some examples, configured to automatically adjust the burst duration.

The controller may be configured to adjust a pulse width of the first train of sub-microsecond pulses and a pulse width of the second train or sub-microsecond pulses. The controller may be configured so that the features/parameters (e.g., pulse width, amplitude, burst duration, etc.) of the first and second trains may be adjusted together.

Any of these systems may include additional pairs of electrodes that may be configured to be operated with the same ground or targeting electrode. For example, any of these systems may include a fourth output coupled to a fourth electrode and a fifth output coupled to a fifth electrode. The controller may be configured to apply the first train of sub-microsecond unipolar pulses to both the first output and the fourth output and to apply the second train of sub-microsecond unipolar pulses to both the second output and the fifth output.

The first electrode, the second electrode, the fourth electrode and the fifth electrode may be part of an applicator. For example, the first electrode, the second electrode, the fourth electrode and the fifth electrode may be part of a single applicator and may be arranged in a plane.

Also described herein are apparatuses for performing the methods described, and the use of such apparatuses for treating cells and/or tissues. These methods and apparatuses may be useful for electroporation (e.g., for transferring material into, or optionally out of, a cell or tissue) and/or for selectively destroying (e.g., non-thermally ablating) cells and/or tissue. These methods and apparatuses may be useful for selectively stimulating (e.g., depolarizing) cells or tissue, including for therapeutic purposes. For example, the apparatuses and methods described herein may be used for therapeutic applications, including, but not limited to, pain control, nerve and/or muscle excitation, gene electrotransfer, activation of immune or endocrine cells, targeted ablation of tumors, etc. In some examples, these methods and apparatuses may be used for treatment of neurologic or physiological disorders, e.g., Parkinson's disease, cardiac ablation, etc. The apparatuses and method described herein may also be used in non-therapeutic applications, including, for example, electroporation.

In particular, the apparatuses and methods described herein can be used to deliver treatment to a specific targeted area of tissue, or a group of cells isolated from a tissue or within a tissue, while avoiding or reducing unintended effects to other non-targeted areas. This may reduce unintended adverse side effects. The apparatuses and methods described herein can also be used to deliver electrostimulation treatments at greater depths that other treatment techniques, including other summation techniques, and may allow greater control of the treatment depth, than conventional electrostimulation treatment apparatuses and methods.

All of the methods and apparatuses described herein, in any combination, are herein contemplated and can be used to achieve the benefits as described herein.

### BRIEF DESCRIPTION OF THE DRAWINGS

A better understanding of the features and advantages of the methods and apparatuses described herein will be obtained by reference to the following detailed description that sets forth illustrative embodiments, and the accompanying drawings of which:
FIGS. 1A-1C depicts the delivery of focal stimulation using an example arrangement of three electrodes in a triangular configuration.
FIG. 2A shows another example of the electrical field in a three-electrode arrangement similar to that shown in FIGS. 1A-1C. FIG. 2B illustrates an example showing focal stimulation using the electrode arrangement of FIG. 2A.
FIGS. 3A-3B depict the delivery of focal stimulation using an example arrangement of five electrodes in a pyramid configuration.
FIG. 4 shows an example of focal stimulation delivered by a 3- electrode arrangement described herein.
FIG. 5 illustrates bipolar cancellation and summation using concurrent pulse trains of sub-microsecond unipolar pulses from a pair of electrodes and a ground electrode as described herein.
FIGS. 6A-6C are examples illustrating the effect of pulse shape of the sub-microsecond unipolar pulses described herein.
FIGS. 7A-7C illustrate examples of angles between the electric field vectors between the first and second electrodes and the ground electrode. FIG. 7A shows a 180 degree angle, in which the ground electrode is in line with the first and second electrodes. FIG. 7B shows an approximately 100 degree angle between the field vectors from first and second electrodes and the ground electrode. FIG. 7C shows an example of a 30 degree angle between the field vectors from the first and second electrodes and the ground electrode.
FIG. 7D illustrates an example of bipolar cancellation between the first and second electrodes and summation at a ground electrode as described herein.
FIG. 7E is a graph showing the effect of the angle between the field vectors of the sub-microsecond unipolar pulses emitted by the first and second electrodes and the ground electrode.
FIG. 8A shows an example of an experimental arrangement using hippocampal neurons and focal stimulation as described herein.
FIG. 8B illustrates the sub-microsecond unipolar pulses applied by the first and second electrodes in the configuration of FIG. 8A as well as an output of the resulting unipolar pulse in a hippocampal cell adjacent to the ground electrode.
FIG. 8C shows an example of a hippocampal cell loaded with a voltage-sensitive dye to show the triggering of action potentials when focally stimulating as described herein.
FIGS. 8D-8G illustrate traces showing action potential initiation based on the voltage-sensitive dye.

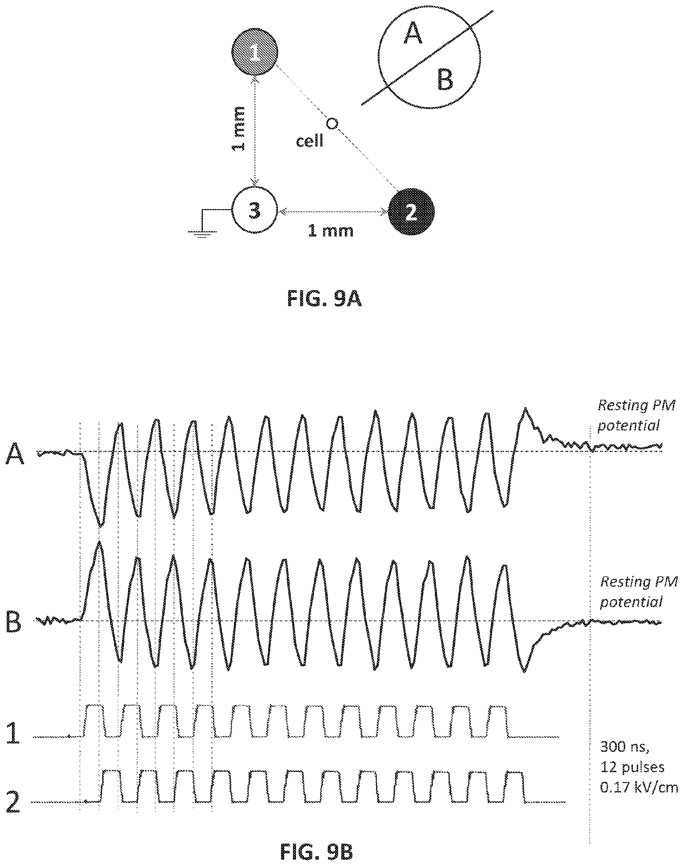

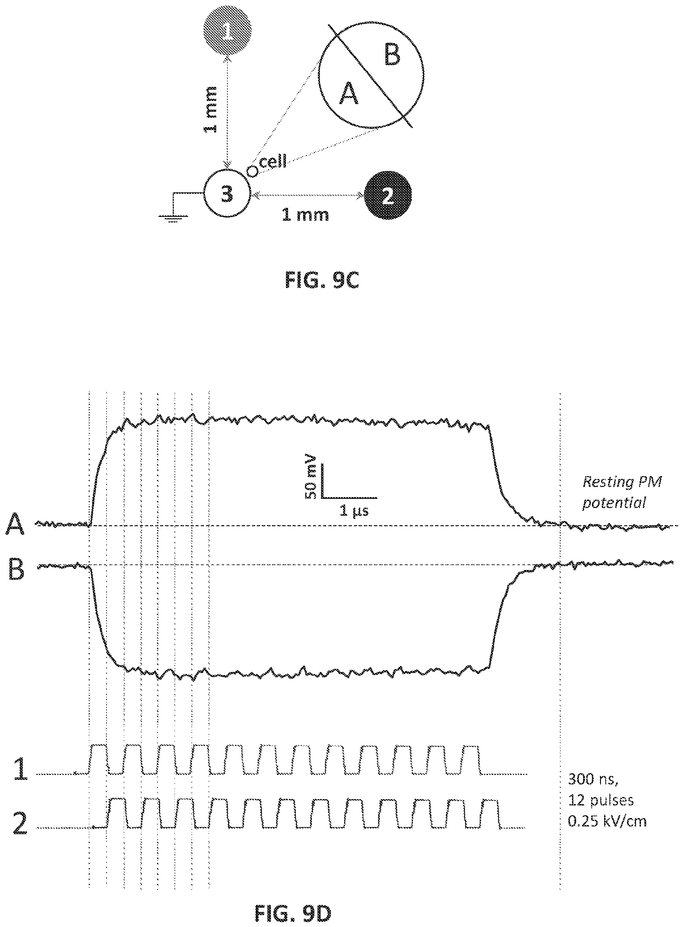

FIGS. 9A-9C illustrate another example of transmembrane potential changes in a model cell line (CHO cells) loaded with voltage-sensitive dye FluoVolt during sub-microsecond exposure. FIG 9A shows a first experimental setup with the cell in-line between the first and second electrode. FIG. 9B shows a first set of traces from the cell during the application of energy with the experimental set-up shown in FIG. 9A. FIG. 9C shows an example of a second experimental set-up with the cell adjacent to the ground electrode. FIG. 9D shows a second set of traces from the cell during the application of energy with the experimental set-up shown in FIG. 9C.
FIG. 10 illustrates one example of a system as described herein.

### DETAILED DESCRIPTION

The present disclosure describes apparatuses (e.g., devices and systems) and non-claimed methods to apply energy selectively to targeted tissue (e.g., tissue region) and/or cell(s) to modify the target tissue and/or cell, while avoiding or reducing substantially modifying non-target tissue and/or cell. The methods and apparatuses described herein can be used to modify the tissue or cell(s) in a desired manner, including ablating, electroporating, stimulating. For example, these methods and apparatuses may be used for targeted nerve and muscle excitation, activation of immune and endocrine cells, cell differentiation, electroporation, cortical stimulation (e.g., from the surface of the skull), cardiac pacing, defibrillation, muscle training and rehabilitation, targeted tumor ablation, gene electro-transfer, pain control, alleviation of Parkinson disease symptoms, diagnosis and/or treatment of neuromuscular and psychiatric disorders, etc. While all descriptions refer to sub-microsecond pulses, it should be understood that all of the methods and apparatuses described herein may be used with 10 microseconds or less. Therefore, the term "sub-microsecond" pulses as used in the present disclosure shall mean a pulse duration of approximately 10 microseconds or less.

Described herein are apparatuses and methods to deliver energy selectively and in a localized manner to a targeted tissue areas and/or cell, including deep tissue targets. In general, these methods and apparatuses may concurrently apply a first train of sub-microsecond unipolar pulses from a first electrode and a second train of sub-microsecond unipolar pulses from a second electrode, wherein the first and second trains of sub-microsecond unipolar pulses are out of phase, for example, 170 degrees out of phase, 190 degrees out of phase, etc. (preferably, 180 degrees out of phase) so that the first and second trains sum at the target tissue or cell that are positioned adjacent (for example, within millimeters and up to several centimeters) to the ground electrode, and cancel at regions near and between the first and second electrodes. The sub-microsecond (e.g., nanosecond) pulses between the first and second electrode form bipolar pulses that prevent the accumulation of charge and therefore prevent depolarization or activation of cells. The sub-microsecond (e.g., nanosecond) electric pulses cancel their stimulatory effect in non-target tissue areas because the electrodes applying the pulsed stimulation that is 180 degrees out of phase results in a reversal of the stimulus polarity, which may be referred to herein as bipolar cancellation. This is described in greater detail below. Because the energy applied between the first and second electrodes is effectively reversing direction during the application of the first and second pulse trains, the apparent electric field between the first and second electrodes is a bipolar electric field that is created by the applied unipolar pulses. In contrast, at or near the ground electrode, which may be located away from the first and second electrodes, for example, in a triangular configuration, may instead sum the applied energy in a comet-shaped region around the ground electrode.

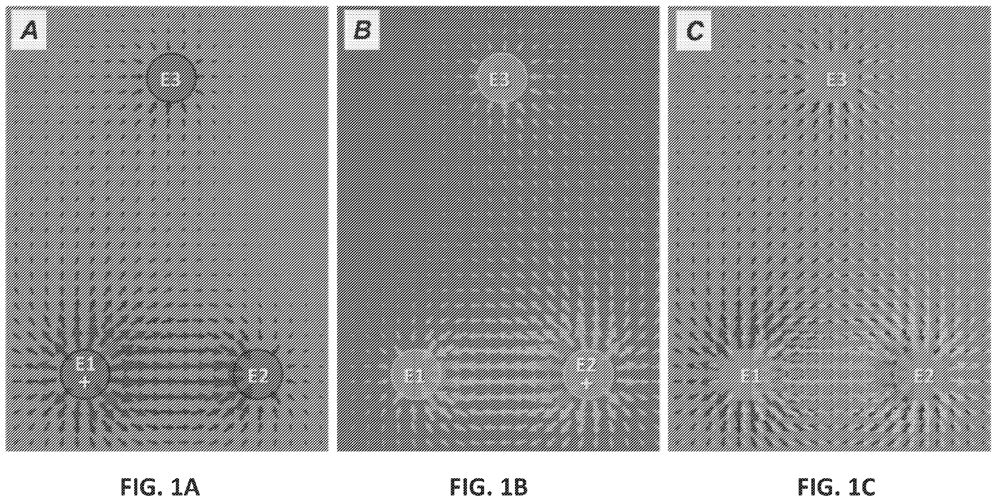

FIGS. 1A-1C illustrates three different states of an example arrangement of three electrodes (E1, E2, and E3) that are configured to deliver electric treatment. E3 is a ground electrode. In particular, the electrodes E1-E3 are configured to focally deliver energy to a targeted tissue area, while avoiding stimulation of untargeted tissue areas. In some examples the electrodes E1-E3 may be all non-penetrating (e.g., skin surface or tissue surface) electrodes. In some examples the electrodes E1-E3 may be all tissue-penetrating electrodes, such as needle electrodes. In some examples the electrodes E1-E3 may be a combination of non-penetrating (e.g., surface) electrodes and penetrating (e.g., needle, blade, knife, etc.) electrodes. For example, in some examples the electrodes E1 and E2 are tissue-penetrating electrodes and electrode E3 is a surface electrode. In another examples, the electrodes E1 and E2 are surface electrodes and electrode E3 is a tissue-penetrating electrode.

In some examples, the three electrodes E1-E3 are arranged in a triangular configuration. In the depicted arrangement, the three electrodes E1-E3 are arranged to define an isosceles triangle. That is, the distance between E1 and E3 is essentially equal to the distance between E2 and E3. In some embodiments, the three electrodes E1-E3 are arranged to define an equilateral triangle, or any other triangle.

The spacing distances between the electrodes E1-E3 can be any suitable distances. In general, when the electrodes E1-E3 are spaced farther apart from each other, a higher level of stimulation energy (e.g., higher voltage and/or current) may be required to achieve a desired therapeutic effect, and the area of tissue affected will be larger. Conversely, when the electrodes E1-E3 are spaced closer to each other, a lower level of stimulation energy can be delivered to achieve a desired therapeutic effect, while the area of tissue affected may be smaller. In some examples, the spacing between the electrodes E1-E3 can be in a range of 0 mm to 10 mm, or 2 mm to 12 mm, or 4 mm to 20 mm, or 6 mm to 30 mm, or 8 mm to 40 mm, or 2 mm to 20 cm, or 4 mm to 10 mm, without limitation. Any suitable electrode spacing distances can be used. In the depicted arrangement of the electrodes E1-E3, the electrodes E1 and E2 are spaced 5 mm apart from each other, the electrodes E1 and E3 are spaced 7 mm apart from each other, and the electrodes E2 and E3 are spaced 7 mm apart from each other.

The trains of unipolar pulses may be delivered by electrodes E1 and E2 in accordance with a coordinated pattern. When applying trains of pulses that are 180 degrees out of phase from E1, the nanosecond electric pulse delivered from electrode E1 while electrode E2 acts as a return (ground) electrode; E3 is fixed as a ground electrode. Conversely, when a nanosecond electric pulse is delivered by electrode E2, electrode E1 acts as a return electrode. Electrode E3 always acts as a return (ground) electrode. That is, electrode E3 acts as a return (ground) electrode while the sub-microsecond pulses are delivered by electrode E1 and while sub-microsecond pulses are delivered by electrode E2. Electrode E3 may be referred to as a targeting electrode. The electric field lines from either active electrode E1 or E2 converge on the targeting electrode, and may merge into a single long pulse. In other words, the electric field direction between the electrodes at the base of the triangle changes by 180°, but this angle may decrease to approximately 0° adjacent to the targeting electrode as the distance between the ground (targeting) electrode and the first and second electrodes increases. As a result, cells situated near and between the two alternately energized electrodes experience bipolar electric field oscillations, while cells close to the targeting electrode may see only a small change of the electric field vector. A train of unipolar pulses applied alternately to the base electrodes (e.g., E1 and E2) therefore generates a long unipolar pulse near the targeting electrode E3, with duration equal to the train duration, as described in more detail below.

This offset interposing of the unipolar, 180 degree out-of-phase, sub-microsecond pulses from the electrodes E1 and E2 results in individual electrical pulses delivered to the tissue that originate according to an example pattern of: a first pulse from E1, then a first pulse from E2, then a second pulse from E1, then a second pulse from E2, and so on. Accordingly, it can be said that electrodes E1 and E2 take turns delivering individual pulses of the sub-microsecond duration. When the alternating trains of sub-microsecond pulses delivered from each of electrodes E1 and E2 have a duty cycle of 50%, the sub-microsecond pulses from the electrodes E1 and E2 collectively merge so that the tissue around the E3 electrode is constantly receiving electrical stimulation (originating either from electrode E1 or from electrode E2). However, the electric field vectors of the electrical pulses from electrode E1 and from electrode E2 have much different directions (as can be visualized by a comparison of FIG. 1A and FIG. 1B).

FIG. 1A depicts electric field when electrode E1 is energized. While the pulse is delivered from electrode E1, electrodes E2 and E3 are acting as return electrodes. The energized electrode is denoted by a "+" sign. The black arrows depict the direction and strength of the electric field in the tissue that is generated by the electrical pulse from the electrode E1. It can be seen that the vectors of the electric field generated by the electrical pulse from the electrode E1 are directionally biased toward the electrodes E2 and E3. That is the case because the electrodes E2 and E3 act as return electrodes when the electrical pulse is delivered from the electrode E1

FIG. 1B depicts a symmetrical situation when E2 is energized. While the pulse is delivered from electrode E2, electrodes E1 and E3 are acting as return electrodes. The light arrows depict the direction and strength of the electric field in the tissue that is generated by the electrical pulse from the electrode E2. It can be seen that the vectors of the electric field generated by the electrical pulse from the electrode E2 are directionally biased toward the electrodes E1 and E3. That is the case because the electrodes E1 and E3 act as return electrodes when the electrical pulse is delivered from the electrode E2.

FIG. 1C depicts an overlap of FIG. 1A and 1B. In effect, FIG. 1C illustrates the combined effects of the interposed trains of sub-microsecond pulses delivered from electrodes E1 and E2 (where individual pulses are delivered from electrodes E1 and E2 in an alternating fashion as described above). It can be seen that the vectors of the electric fields generated from the trains of sub-microsecond pulses delivered from electrodes E1 and E2 are in opposite directions in some areas of the tissue. For example, in the tissue areas in between and around the electrodes E1 and E2, the electric field generated from the trains of sub-microsecond pulses delivered from electrodes E1 and E2 are in opposite directions. The opposing electric field directions prevents charge accumulation on the cell membrane in these regions and prevents or cancels bioeffects of pulses.

However, in the tissue area near the electrode E3, the electric field generated from the trains of sub-microsecond pulses delivered from electrodes E1 and E2 may be in generally similar direction. In that tissue area, there is no cancellation effect, but instead may result in summation.

The applied sub-microsecond pulses (e.g., nanosecond electric pulses (nsEP), picosecond pulses, etc.) may be delivered to provide bioeffects as described herein and can have various parameters. For example, the sub-microsecond pulses are unipolar and can have various pulse widths/durations, amplitudes, pulse trains parameters, and so on. In some examples the individual pulses are in the nanosecond range (e.g., 1 ns to 999 ns) for width/duration, without limitation. In some examples the individual pulses have an amplitude of less than 1 kV, or greater than 1 kV, without limitation.

For a biological target (e.g., a cancer cell, or a neuron, or any live cell), individual sub-microsecond pulses delivered from electrodes E1 and E2 may effectively "merge" in the tissue area near electrode E3 into one long pulse whose duration is equal to the total duration of pulse trains applied to electrodes E1 and E2. Such a long pulse is efficient at lower thresholds, and causes stronger effects above the threshold, as compared to either an individual electrical stimulation pulse or a train of sub-microsecond pulses applied to the tissue area near electrode E3. In contrast, cells situated near electrodes E1 and E2 will experience bipolar sub-microsecond pulses, which have higher thresholds and cause much less biological effects. With a proper choice of pulse parameters, there is no charge accumulation on cell membranes of cells situated near electrodes E1 and E2, even if the sub-microsecond pulses are applied for indefinitely long time. The cells situated near electrodes E1 and E2 remain under a threshold for biological effect. In contrast, the cells situated near electrode E3 experience a one polarity extra-long stimulating pulse near electrode E3. As a result, little or no bioeffects are produced in the tissue near electrodes E1 and E2, and maximum effects are produced in the tissue near electrode E3.

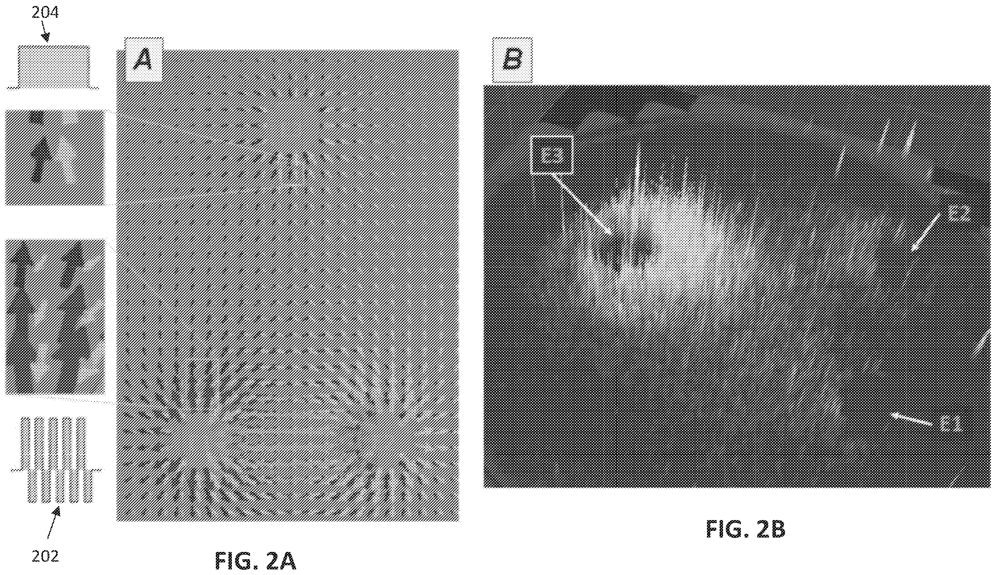

FIGS. 2A-2B provide additional information and clarification regarding the operation and effects of the three electrodes E1-E3 that are arranged in a triangular configuration as in FIGS. 1A-1C. In general, the bioeffects will gradually decrease from the area around electrode E3, in the direction towards electrodes E1 and E2, in a comet-like shape. Placing electrodes E1 and E2 closer to each other may make the tail of the comet-like shape narrower and longer, i.e., will enable a deeper penetration of the focal stimulation.

FIG. 2B shows a three-dimensional spatiotemporal sub-microsecond pulse summation that represents the effects of the three electrodes E1-E3 that are arranged in a triangular configuration (as in FIGS. 1A-1C). In particular, this example illustrates an electroporation effect when trains of five 600-ns stimuli at 4 kV and 50% duty cycle were applied in an interposed fashion (alternating) to electrodes E1 and E2. The locations of the electrodes E1-E3 are indicated by the arrows. It can be seen that a strong electroporation effect is observed near electrode E3, but not near electrodes E1 or E2. In this manner, a particular tissue area can be targeted for treatment, while untargeted tissue areas can be free from unintended effects of the treatment.

The electric field vector map shown in FIG. 2A is the same as FIG. 1C, with the addition of two magnifying boxes for two example regions (one region near electrode E1 and a second region near electrode E3). These magnifying boxes illustrate the change of the electric field vectors in opposing directions near electrode E1, and just minimal directional changes near electrode E3. The electric stimuli experienced by cells situated in these areas (e.g., a series of asymmetrical bipolar sub-microsecond pulses 202 near electrode E1, and the equivalent of one long unipolar stimulus 204 near electrode E3) are shown in graphical format next to the magnifier boxes.

In FIG. 2B, a train of five 600-ns pulses at 4 kV was applied in alternation to electrodes E1 and E2, as described above. The stimulation intensity was selected to produce electroporation near electrode E3, with minimal effects near electrodes E1 and E2. Electroporated cells were detected by the uptake of a cell-impermeable fluorescent dye YO-PRO-1. FIG. 2B shows that, as expected, most electroporation occurred in a comet-like shape extending from electrode E3 towards electrodes E1 and E2.

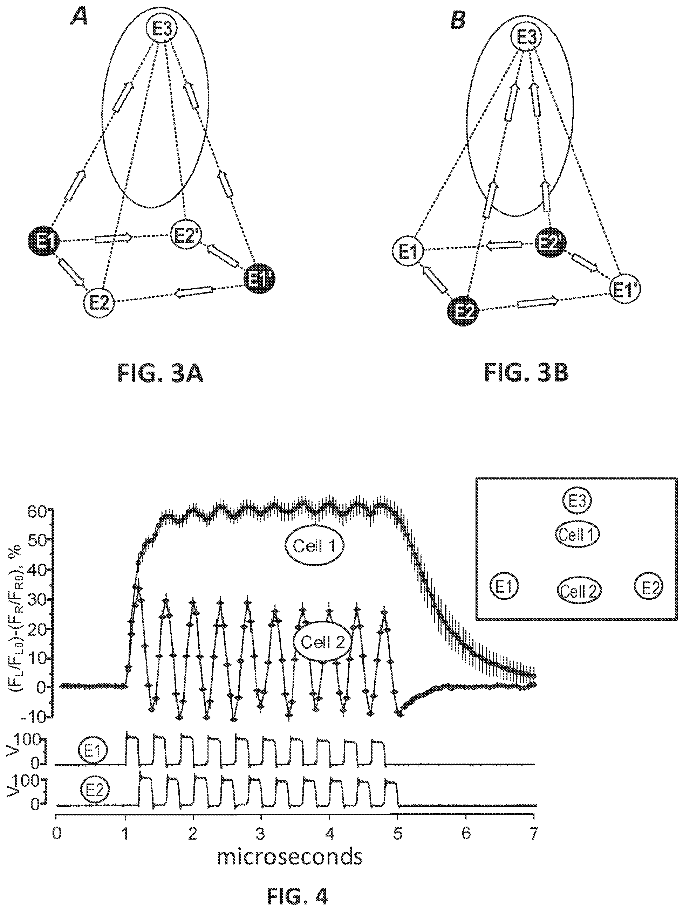

FIGS. 3A and 3B shows another example arrangement of electrodes that can be used to deliver targeted electric stimuli to tissue. This depicts two different states of an example arrangement of five electrodes (E1, E1', E2, E2', and E3) that are configured to deliver electric treatment. In particular, the electrodes E1-E3 are configured to deliver focal stimulation to a targeted tissue area (e.g., as depicted by the shaded ellipsoidal area), while avoiding stimulation of untargeted tissue areas, as described further below. E3 may be configured as a permanent ground electrode (e.g., targeting electrode).

In some examples, the electrodes of the pyramid are all non-penetrating (e.g., skin surface or tissue surface, or plate) electrodes. In some embodiments, the electrodes may be all tissue-penetrating electrodes, such as needle electrodes. In some embodiments, the electrodes may be a combination of surface electrodes and tissue-penetrating electrodes. For example, in some embodiments the electrodes E1, E1', E2, and E2' are tissue-penetrating electrodes and electrode E3 is a tissue surface electrode. In another embodiment, the electrodes E1, E1', E2, and E2' are tissue surface electrodes and electrode E3 is a tissue-penetrating electrode. Any other combination is within the scope of the present disclosure.

In the depicted embodiment, the five electrodes E1-E3 are arranged in a pyramidal configuration (with electrode E3 at the apex of the pyramid). In the depicted arrangement, the four electrodes E1, E1', E2, and E2' that define the base of the pyramid are arranged to define a quadrilateral. The quadrilateral can be a square, a rectangle, a trapezoid, a rhombus, a parallelogram, or any other type of quadrilateral shape. The shape and size of the pyramid base can be selected to create a tissue treatment area having a desired shape, size, and depth.

The spacing distances between the electrodes E1-E3 can be any suitable distances. In general, when the electrodes E1-E3 are spaced farther apart from each other, a higher level of stimulation energy (e.g., higher voltage and/or current) will be required to achieve a desired therapeutic effect, and the area of tissue affected will be larger. Conversely, when the electrodes E1-E3 are spaced closer to each other, a lower level of stimulation energy can be delivered to achieve a desired therapeutic effect, while the area of tissue affected will be smaller. In some example embodiments, the spacing between the electrodes E1-E3 can be in a range of 0 mm to 10 mm, or 2 mm to 12 mm, or 4 mm to 20 mm, or 6 mm to 30 mm, or 8 mm to 40 mm, or 4 mm to 10 mm, 4 mm to 40 mm, or 6 mm to 60 mm, or 1cm to 20 cm, without limitation. Any suitable electrode spacing distances can be used.

The electrodes that form the base of the pyramid may be energized in pairs. That is, each electrode E1 and E1' is energized to each deliver a pulse at the same time (as illustrated in FIG. 3A), and each electrode E2 and E2' is energized to each deliver a pulse at the same time (as illustrated in FIG. 3B).

As shown in FIG. 3A, when the electrodes E1 and E1' are concurrently energized, the electrodes E2 and E2' are concurrently acting as return/ground electrodes (along with electrode E3). As shown in FIG. 3B, when the electrodes E2 and E2' are concurrently energized, the E1 and E1' are concurrently acting as return electrodes (along with electrode E3). Electrode E3 always acts as a return (ground) electrode.

In some embodiments, the electrode pairs are used to deliver interposed sub-microsecond pulses repeatedly at the duty cycle of 50% (e.g., in the manner described above regarding the triangular arrangement of the electrodes E1-E3). Such a pyramid configuration of the electrodes E1-E3 further decreases the electric field and current density at the tissue region near the four electrodes E1, E1', E2, and E2' that define the base of the pyramid, and further enhances focal stimulation at the tissue region near electrode E3 (at the apex of the pyramid). Accordingly, a desired three-dimensional shape of targeted tissue can be obtained. In the depicted embodiment, the three-dimensional shape of targeted tissue comprises an ellipsoid.

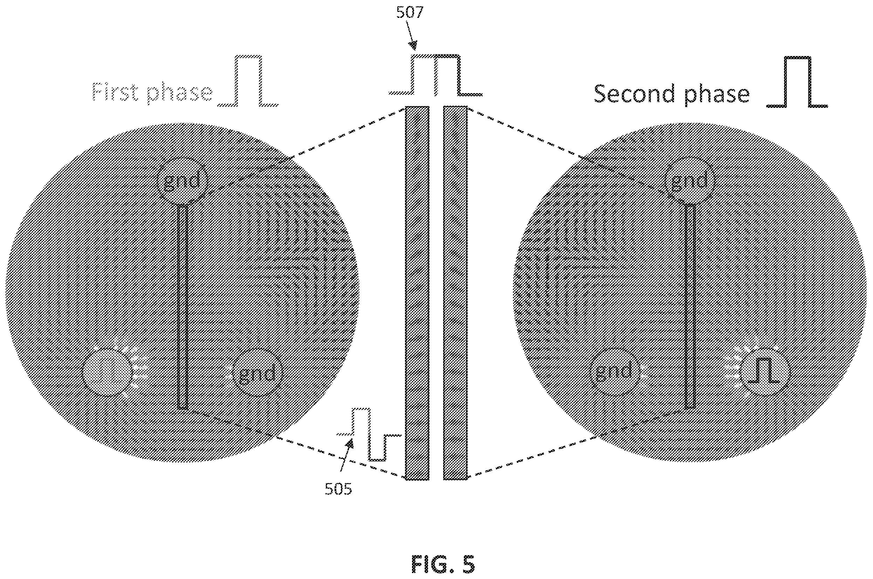

FIG. 5 illustrates computer simulation data for another example of the cancellation effect discussed above. In FIG. 5, the left side image shows a first electrode (applying a unipolar pulse train as described above) in a first phase, during which the third (permanent ground electrode) and the second electrode operate as ground electrodes. The right side of the image shows an example of a second phase, during which the second electrode applies a monopolar pulse identical (but 180 degrees out of phase) of the pulse previously applied by the first electrode. The columns between the two images shows the direction of the electrical field energy in a region between the first and second electrodes, extending up to the third electrode. Because of the change in direction of the field between when the energy is applied by the first electrode and the second electrode, the resulting pulse either looks like a bipolar pulse 505, in the region immediately between the first and second electrode, or a longer pules 507, in the region closer to the third electrode and away from the region immediately between the first and second electrodes.

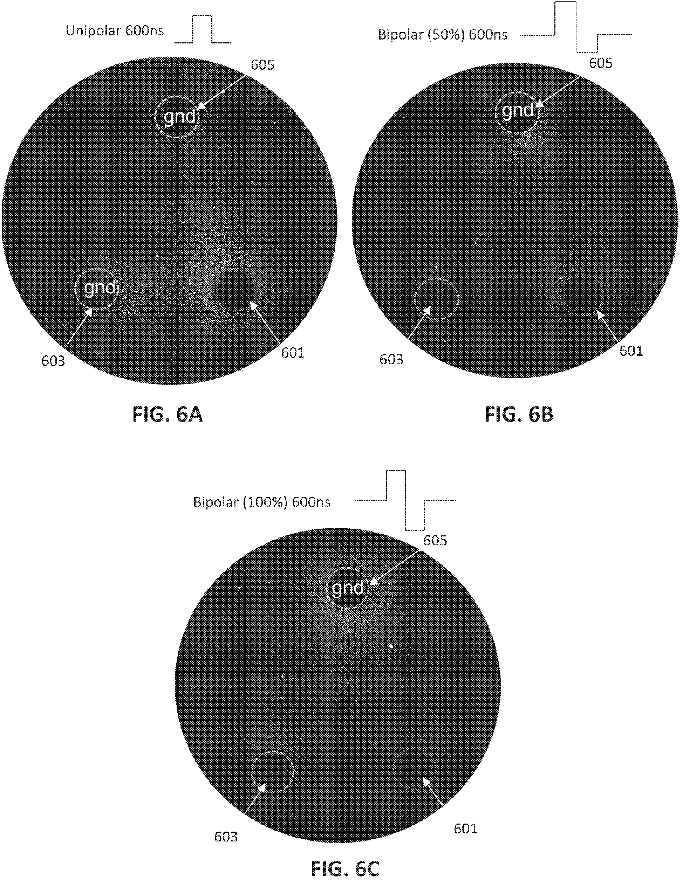

In general the train of sub-microsecond unipolar pulses emitted by the first and second electrodes may be approximately the same size and shape. For example, FIGS. 6A-6C illustrate the effect of different sizes or shapes for the emitted pulses on the ability to modify target vs. non-target tissue or cells. For example, in FIG. 6A the train of sub-microsecond unipolar pulses is emitted just from the first electrode 601, but not from the second electrode 603. As shown, applying just a train of sub-microsecond unipolar pulses from the first electrode without applying the 180 degrees out of phase train of sub-microsecond unipolar pulses from the second electrode results in modifying cells around the first electrode, and between the first and second electrode in a somewhat diffuse, non-targeted manner. In FIG. 6B a first train of sub-microsecond unipolar pulses having a first amplitude is emitted from the first electrode, while a second train of sub-microsecond unipolar pulses having a second amplitude that is approximately half the amplitude of the first train of sub-microsecond unipolar pulses results in directing the energy toward the third (targeting or ground) electrode 605. The tail of the comet-shaped affected (target) region extends towards the first electrode. In contrast, FIG. 6C shows an example in which the first 601 and second 603 electrodes both apply a train of sub-microsecond unipolar pulses having equivalent amplitude. Virtually all of the modified target tissue or cell(s) is in a comet-shaped region around the third (targeting or ground) electrode 605. In FIGS. 6A-6C a voltage-sensitive dye may be used to show an effect (e.g., depolarization) on the target or non-target tissue or cells.

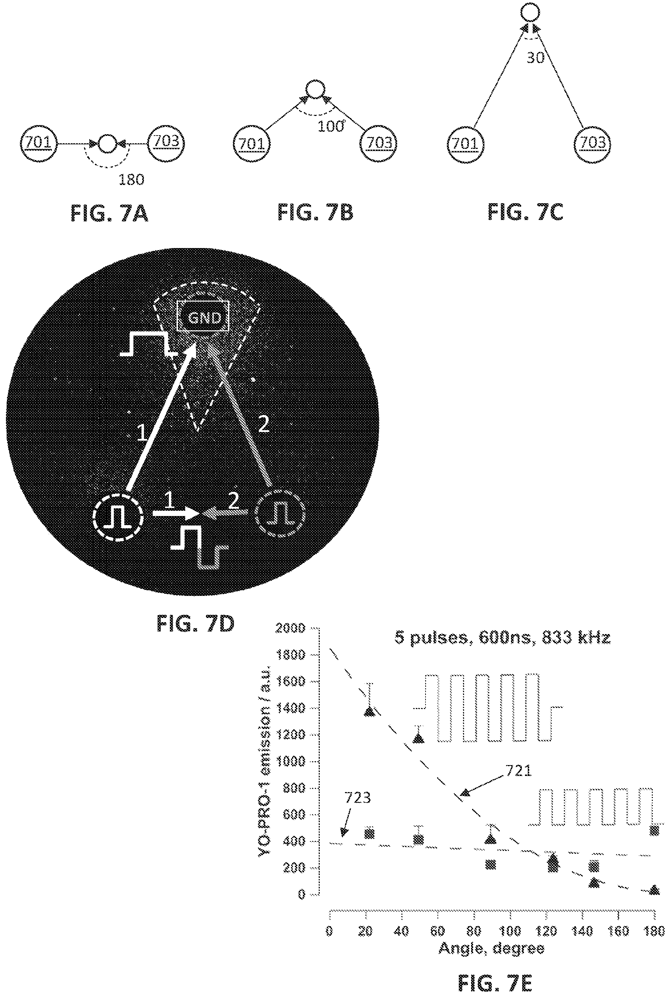

The shape of the region of effect may be modified by changing the amplitude and/or arrangement of the first and second electrodes relative to the third (ground) electrode, as shown in FIGS. 7A-7E. For example, FIGS. 7A-7C illustrate examples of different angles of the applied electrical energy from a first electrode 701 and a second electrode 703 (each of which emit a train of sub-microsecond unipolar pulses) and a ground (or targeting) electrode. In FIG. 7A the angle between the electric field vectors when the electrodes are in-line is 180 degrees. In FIG. 7B the ground electrode is offset from the distance between the first and second electrodes so that the angle is approximately 100 degrees. In FIG. 7C the ground electrode is separated from the first and second electrodes by a larger distance, and the angle between the electric field vectors is approximately 30 degree.

FIG. 7D shows an example of a result of applying a first train of sub-microsecond unipolar pulses from a first electrode and a second train of sub-microsecond unipolar pulses from a second electrode, wherein the first and second trains of sub-microsecond unipolar pulses are concurrently applied and are 180 degrees out of phase. As shown, this results in significant activation of target tissue (or cells) adjacent to the ground electrode. This experimental setup was used to adjust the position of the ground electrode relative to the first and second electrodes and therefore the angle between the electric field vectors of the first and second electrodes and the ground (targeting) electrode.

In this example, the angle between the electric field vectors of two sub-microsecond pulses delivered one after another determined whether the effect is canceled (with the vectors pointing in the opposite directions) or enhanced (when the vectors pointed in the same direction). As shown in the graph of FIG. 7E, for an example such as described above 721, the maximum bipolar cancellation (13-fold vs a unipolar pulse) was observed with 180° vector change. Cancellation tapered out towards the targeting electrode because of the reduction of the vector change angle. At angles less than 90-120°, cancellation was replaced by summation, i.e., the effects became stronger (3-fold at 22°). In this graph, the second line 723 shows the effect of angle when only one of the first or second electrodes applied a train of pulses (similar to FIG. 6A).

The vector angle change on the efficiency of electroporation using 600-ns pulses in bovine pulmonary artery endothelial cell monolayers showed a maximum bipolar cancellation (13-fold vs a unipolar pulse) was observed with 180° vector change. Cancellation tapered out towards the targeting electrode because of the reduction of the vector change angle. At angles less than 90-120°, cancellation was replaced by summation, i.e., the effects became stronger (3-fold at 22°). In another experiment, this effect was utilized to evoke electroporation and excitation (measured by Ca²⁺ mobilization) in smooth muscle cell monolayers. The effect was achieved at the targeting electrode while avoiding it at two base electrodes, despite the stronger electric field there. Indeed, bipolar nanosecond oscillations between alternately energized electrodes had low biological efficiency (because of both the nanosecond duration and bipolar pulse shape). The same pulses merged into a long unipolar pulse near the targeting electrode, and the duration of this created unipolar pulse can be made in µs or ms range, making it far more efficient than the bipolar nanosecond oscillations. As stated above, this effect can be utilized for enhancing focal stimulation (e.g., electroporation) at one of the electrodes while fully avoiding it at the other electrodes.

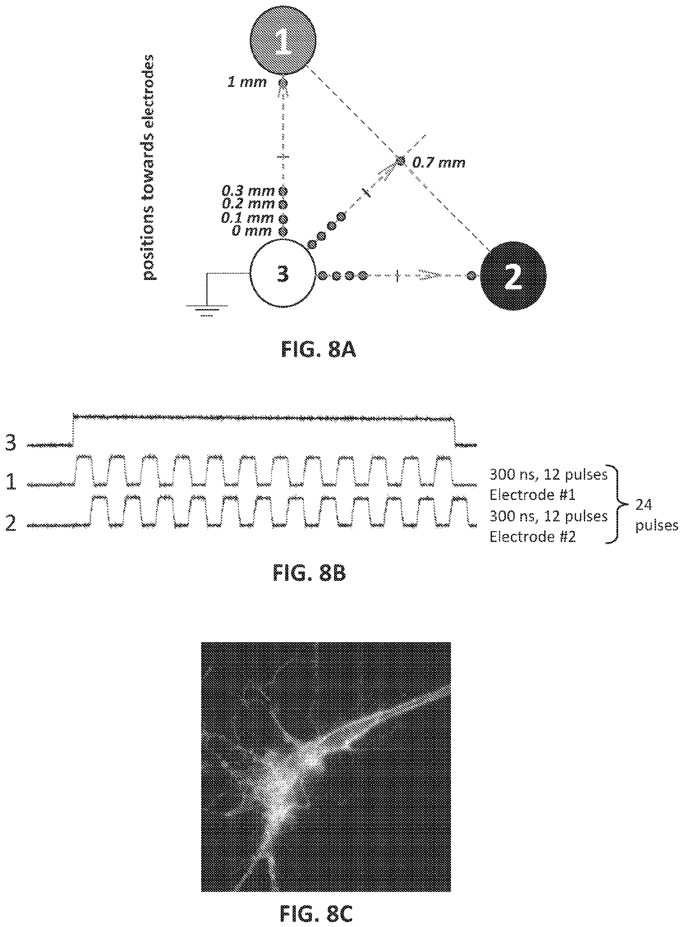

In a further experiment, the bipolar cancellation phenomenon was used in a 3-electrode array to trigger action potentials (APs) in dissociated hippocampal neurons by spatiotemporal summation of sub-microsecond pulses at a chosen location. Neurons were enzymatically isolated from newborn mice and incubated in glass-bottomed culture dishes for 2-3 weeks until they started producing APs. To visualize plasma membrane (PM) electric potential changes, neurons were loaded with a FluoVolt voltage-sensitive dye. Time-lapse image stacks (3048 frames/s) were acquired using a CCD Camera on an inverted microscope. The electrode array for neuron excitation constituted three needles in the isosceles right triangle positioned 50 µm above a culture dish perpendicular to the bottom. A bipolar electric field was created by alternating twenty-four 300 ns unipolar electric pulses between two electrodes where one electrode served as a ground at the moment when the other was energized. Both active electrodes were distanced 1mm apart from the third, permanently a ground one where electric field lines converged, combining into a single long pulse, as shown in FIG. 8A. Such exposure aimed to use bipolar cancellation to suppress neuron excitation anywhere within the array except for the targeted region at the ground electrode.

FIGS. 8A-8G illustrate the effect of relative proximity to the ground electrode when applying a first train of sub-microsecond unipolar pulses from a first electrode and a second train of sub-microsecond unipolar pulses from a second electrode, wherein the first and second trains of sub-microsecond unipolar pulses are concurrently applied and are 180 degrees out of phase. FIG. 8A shows the experimental set-up, described above, showing the position of the first electrode (1), the second electrode (2), and the ground electrode (3), as well as example spacings and positions of the target cells, which in this example were hippocampal neurons. Changes in electric field strength at different distances from the ground electrode were accounted for using low-frequency finite element solver Sim4Life V5.2. Responsive neurons were exposed to 24, 300 ns pulses (12 from each active electrode) at 1.6 MHz. Pulses were alternating to create a cancellation effect between active electrodes, as described herein. A long effective pulse formed at the ground electrode due to spatiotemporal summation of nanosecond electric pulses.

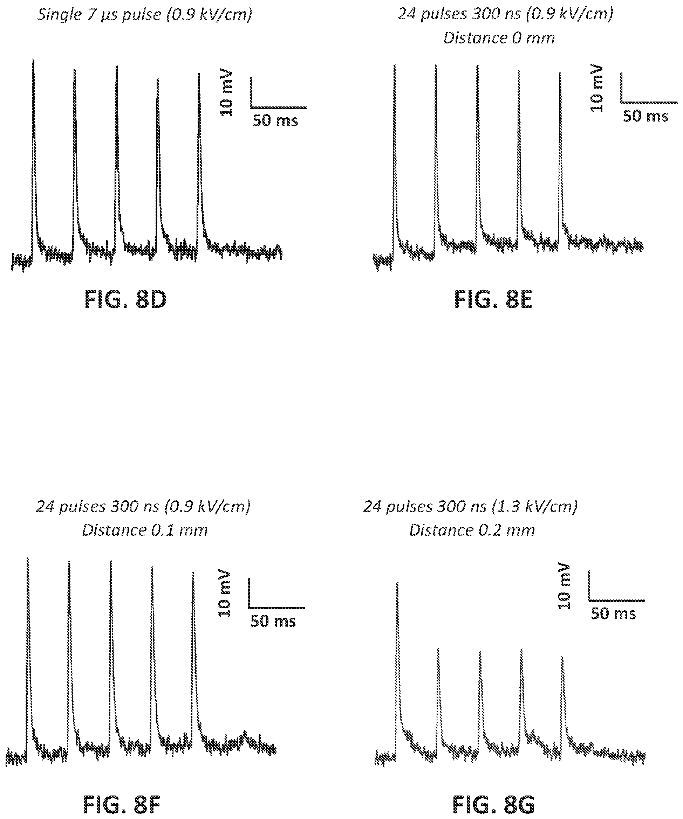

FIG. 8B shows the pulse train applied by the first electrode and the second electrode, as well as the anticipated shape of the resulting long pulse at or adjacent to the third electrode. FIG. 8C shows an example of a hippocampal neuron loaded with the voltage sensitive dye. FIGS. 8D-8G show the results of this experiment. In FIG. 8D, a long (7 µs) pulse at 0.9 kV/cm initiated action potentials (APs) in neurons at any tested distance from ground electrode. In FIG. 8E and 8F, in the range from 0 to 0.1 mm from ground electrode, APs could be initiated by 24 alternating 300 ns pulses at 0.9 kV/cm. In the range to 0.2 mm from the ground electrode, APs could be initiated by trains of 300 ns pulses at a higher threshold of 1.3 kV/cm.

However, in this example, as shown in FIG. 8G, no APs was initiated at distances at or longer than about 0.3 mm from the ground electrode by 24 alternating 300 ns pulses or 12 pulses from electrode 1 or 2 (no cancellation effect) at electric field strengths up to 1.5 kV/cm. Further increase in field strength resulted in electropermeabilization-like responses of these cells.

### FURTHER EXAMPLES

FIGS. 4 and 9A-8D illustrate the specific targeting of cells using the techniques described herein. Fluorescence traces in FIG. 4 reflect the difference in the membrane potential induced at the opposite poles of the cell. It is expressed as the difference in fluorescence between the left and right hemispheric regions of interest as: (F_{L}/F_{L0})-(F_{R}/F_{R0}) * 100%, where F_{L} is the emission intensity in the left hemispheric region of interest; F_{L0} is the starting emission intensity in the left hemispheric region of interest (as averaged from 5 images taken before the delivery of sub-microsecond pulses); and F_{R} and F_{R0} are, respectively, the same measurements for the right hemispheric region of interest of the same cell.

In the "Cell 2" located between electrodes E1 and E2 of FIG. 4, sub-microsecond pulse trains caused periodic charging and discharging of the cell membrane (as illustrated by the zig-zag line on the graph of FIG. 4). In the "Cell 1" near electrode E3, membrane was polarized for the entire duration of the sub-microsecond pulses, with modest oscillations (as illustrated by the upper steady line on the graph of FIG. 4).

For experimental validation of electric field bipolar oscillations, a custom-made strobe imaging system was used to observe changes in the PM electric potential of CHO-K1 cells, which reflected the electric field vector alterations upon exposure. Closer to active electrodes, PM potential at the side of the cell facing either of them alternated from de- to hyperpolarization at 300 ns intervals. Exposure of the cell in a targeted region resulted in a ~7 µs long continuous PM depolarization at the side facing the ground. If above the excitation threshold, this PM depolarization would be sufficient to excite neurons. Neurons were exposed at various distances from the ground to active electrodes to analyze how their position within the electrode array affects AP initiation. The amplitude of the sub-microsecond pulses in the trains was increased until AP was initiated. It was found that APs can be triggered at the same threshold within 100 µm from the ground. In the range from 100 to 300 µm, the AP threshold increased twofold. Farther than 300 µm from the ground, there was no successful AP initiation regardless of pulse amplitude. If neurons were exposed to a 7 µs pulse instead, AP was initiated anywhere within the array. This result shows how the bipolar cancellation phenomenon in the 3-electrode array can be utilized for targeted stimulation at a single electrode.

FIG. 9A shows an example of the first experimental set-up as described. For measurements of the induced membrane potential, the cell is divided into a left (A) hemisphere and a right (B) hemisphere. As discussed above and shown in FIGs. 9A-B, when the cell is positioned between the first and second electrode and is not positioned adjacent to the ground electrode (3), the train of sub-microsecond unipolar pulses does not accumulate the membrane charge. The pulse trains applied by the first and second electrodes are shown (e.g., 300 ns, 12 pulses per train, 0.17 kV/cm).

In contrast, when the cell is positioned adjacent to the ground or targeting electrode (3), as shown in the diagram of FIG. 9C, exposure to 24 alternating 300 ns pulses at 0.25 kV/cm results in 7 µs long depolarization on plasma membrane of CHO cell situated in targeted region nearby permanent ground/targeting electrode. This depolarization was identical to depolarization evoked by 7 µs pulse and can lead to AP initiation if threshold is reached. The traces shown in FIG. 9D illustrate the summation of the electrical field energy.

### Apparatus

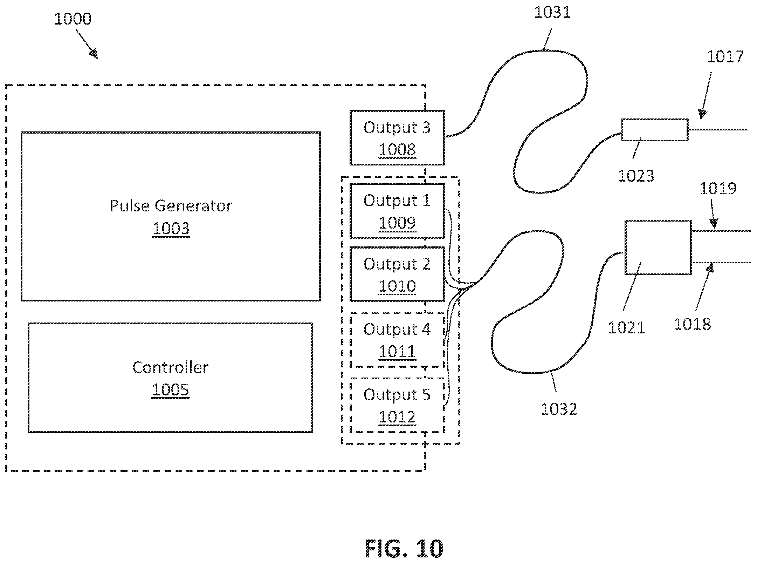

FIG. 10 illustrates one example of an apparatus as described herein. In this example, the system 1000 for applying energy to a target tissue or cell includes a pulse generator 1003 and a controller 1005, and a plurality of electrical connectors (outputs 1008, 1009, 1010, 1011, 1012) that are configured to couple to electrodes. For example, the first output 1009 (output 1) is configured to couple to a first electrode (e.g., 1018) and the second output 1010 (output 2) is configured to couple to a second electrode (e.g., 1019). The third output 1008 (output 3) is configured to couple to a ground electrode (or targeting electrode) 1017. Optional additional outputs, e.g., fourth output 1011 (output 4) and fifth output 1012 (output 5) are also shown, which may couple to additional electrodes (not shown). These outputs may be individually or collectively coupled (e.g., via one or more cables 1031, 1032) to one or more applicators 1021, 1023.

In this example the pulse generator 1003 may be configured to generate (including concurrently) a first train of sub-microsecond unipolar pulses and a second train of sub-microsecond unipolar pulses, wherein the first and second trains of sub-microsecond unipolar pulses are 180 degrees out of phase. The controller 1005 may be configured to communicate with the pulse generator and the outputs to apply the first train or sub-microsecond pulses to the first output concurrently with the second train of sub-microsecond pulses to the second output, and to couple the third output to the ground/targeting electrode. The controller may include circuitry (e.g., memory, one or more processors, communication circuitry, power control circuitry, etc.) and may be part of or separate from the pulse generator. In some examples the controller and pulse generator may be integrated together and may be housed in a common housing. The pulse generator may include one or more inputs for receiving user (control) input, including for adjusting the pulsing parameters, as described above.

The systems described herein may also include one or more applicators. For example, an applicators 1021, 1023 may include all or some of the electrodes. The applicator may be configured to be hand-held and applied to a subject (e.g., patient) by a user. The applicator may be computer-controlled, including robotically controlled. In FIG. 10 two applicators are shown, the first applicator 1021 for the first and second (or more) electrodes and the second applicator 1023 for just the targeting (e.g., ground) electrode. In some examples the electrodes and applicator may be built into a mount or housing, which may include, hold or engage a dish (e.g., multi-well dish, etc.) for holding cells and/or tissue. In some implementations, the applicator may be attached to the robotic or moveable arm.

Examples of the methods of the present disclosure may be implemented using computer software, firmware, or hardware. Various programming languages and operating systems may be used to implement the present disclosure. The program that runs the method and system may include a separate program code including a set of instructions for performing a desired operation or may include a plurality of modules that perform such sub-operations of an operation or may be part of a single module of a larger program providing the operation. The modular construction facilitates adding, deleting, updating and/or amending the modules therein and/or features within the modules.

Certain examples may relate to a machine-readable medium (e.g., computer-readable media) or computer program products that include program instructions and/or data (including data structures) for performing various computer-implemented operations. A machine-readable medium may be used to store software and data which causes the system to perform methods of the present disclosure. The above-mentioned machine-readable medium may include any suitable medium capable of storing and transmitting information in a form accessible by processing device, for example, a computer. Some examples of the machine-readable medium include, but not limited to, magnetic disc storage such as hard disks, floppy disks, magnetic tapes. It may also include a flash memory device, optical storage, random access memory, etc. The data and program instructions may also be embodied on a carrier wave or other transport medium. Examples of program instructions include both machine code, such as produced by a compiler, and files containing higher level code that may be executed using an interpreter.

Any of the methods (including user interfaces) described herein may be implemented as software, hardware or firmware, and may be described as a non-transitory computer-readable storage medium storing a set of instructions capable of being executed by a processor (e.g., computer, tablet, smartphone, etc.), that when executed by the processor causes the processor to perform or control performing of any of the steps, including but not limited to: displaying, communicating with the user, analyzing, modifying parameters (including timing, frequency, intensity, etc.), determining, alerting, or the like. In some exemplary examples, hardware may be used in combination with software instructions to implement the present disclosure.

It should be appreciated that all combinations of the foregoing concepts and additional concepts discussed in greater detail below (provided such concepts are not mutually inconsistent) are contemplated as being part of the inventive subject matter disclosed herein and may be used to achieve the benefits described herein.

The process parameters and sequence of steps described and/or illustrated herein are given by way of example only and can be varied as desired. For example, while the steps illustrated and/or described herein may be shown or discussed in a particular order, these steps do not necessarily need to be performed in the order illustrated or discussed. The various example methods described and/or illustrated herein may also omit one or more of the steps described or illustrated herein or include additional steps in addition to those disclosed.

The term "memory" or "memory device," as used herein, generally represents any type or form of volatile or non-volatile storage device or medium capable of storing data and/or computer-readable instructions. In one example, a memory device may store, load, and/or maintain one or more of the modules described herein. Examples of memory devices comprise, without limitation, Random Access Memory (RAM), Read Only Memory (ROM), flash memory, Hard Disk Drives (HDDs), Solid-State Drives (SSDs), optical disk drives, caches, variations or combinations of one or more of the same, or any other suitable storage memory.

In addition, the term "processor" or "physical processor," as used herein, generally refers to any type or form of hardware-implemented processing unit capable of interpreting and/or executing computer-readable instructions. In one example, a physical processor may access and/or modify one or more modules stored in the above-described memory device. Examples of physical processors comprise, without limitation, microprocessors, microcontrollers, Central Processing Units (CPUs), Field-Programmable Gate Arrays (FPGAs) that implement softcore processors, Application-Specific Integrated Circuits (ASICs), portions of one or more of the same, variations or combinations of one or more of the same, or any other suitable physical processor.

Although illustrated as separate elements, the method steps described and/or illustrated herein may represent portions of a single application. In addition, in some embodiments one or more of these steps may represent or correspond to one or more software applications or programs that, when executed by a computing device, may cause the computing device to perform one or more tasks, such as the method step.

In addition, one or more of the devices described herein may transform data, physical devices, and/or representations of physical devices from one form to another. Additionally or alternatively, one or more of the modules recited herein may transform a processor, volatile memory, non-volatile memory, and/or any other portion of a physical computing device from one form of computing device to another form of computing device by executing on the computing device, storing data on the computing device, and/or otherwise interacting with the computing device.

A person of ordinary skill in the art will recognize that any process or method disclosed herein can be modified in many ways. The process parameters and sequence of the steps described and/or illustrated herein are given by way of example only and can be varied as desired. For example, while the steps illustrated and/or described herein may be shown or discussed in a particular order, these steps do not necessarily need to be performed in the order illustrated or discussed.

The various exemplary methods described and/or illustrated herein may also omit one or more of the steps described or illustrated herein or comprise additional steps in addition to those disclosed. Further, a step of any method as disclosed herein can be combined with any one or more steps of any other method as disclosed herein.

The processor as described herein can be configured to perform one or more steps of any method disclosed herein. Alternatively or in combination, the processor can be configured to combine one or more steps of one or more methods as disclosed herein.

When a feature or element is herein referred to as being "on" another feature or element, it can be directly on the other feature or element or intervening features and/or elements may also be present. In contrast, when a feature or element is referred to as being "directly on" another feature or element, there are no intervening features or elements present. It will also be understood that, when a feature or element is referred to as being "connected", "attached" or "coupled" to another feature or element, it can be directly connected, attached or coupled to the other feature or element or intervening features or elements may be present. In contrast, when a feature or element is referred to as being "directly connected", "directly attached" or "directly coupled" to another feature or element, there are no intervening features or elements present. Although described or shown with respect to one embodiment, the features and elements so described or shown can apply to other embodiments. It will also be appreciated by those of skill in the art that references to a structure or feature that is disposed "adjacent" another feature may have portions that overlap or underlie the adjacent feature.

Terminology used herein is for the purpose of describing particular embodiments only and is not intended to be limiting of the invention. For example, as used herein, the singular forms "a", "an" and "the" are intended to include the plural forms as well, unless the context clearly indicates otherwise. It will be further understood that the terms "comprises" and/or "comprising," when used in this specification, specify the presence of stated features, steps, operations, elements, and/or components, but do not preclude the presence or addition of one or more other features, steps, operations, elements, components, and/or groups thereof. As used herein, the term "and/or" includes any and all combinations of one or more of the associated listed items and may be abbreviated as "/".

Spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if a device in the figures is inverted, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly. Similarly, the terms "upwardly", "downwardly", "vertical", "horizontal" and the like are used herein for the purpose of explanation only unless specifically indicated otherwise.

Although the terms "first" and "second" may be used herein to describe various features/elements (including steps), these features/elements should not be limited by these terms, unless the context indicates otherwise. These terms may be used to distinguish one feature/element from another feature/element. Thus, a first feature/element discussed below could be termed a second feature/element, and similarly, a second feature/element discussed below could be termed a first feature/element without departing from the teachings of the present invention.

In general, any of the apparatuses and methods described herein should be understood to be inclusive, but all or a sub-set of the components and/or steps may alternatively be exclusive, and may be expressed as "consisting of" or alternatively "consisting essentially of' the various components, steps, sub-components or sub-steps.

As used herein in the specification and claims, including as used in the examples and unless otherwise expressly specified, all numbers may be read as if prefaced by the word "about" or "approximately," even if the term does not expressly appear. The phrase "about" or "approximately" may be used when describing magnitude and/or position to indicate that the value and/or position described is within a reasonable expected range of values and/or positions. For example, a numeric value may have a value that is +/- 0.1% of the stated value (or range of values), +/- 1% of the stated value (or range of values), +/- 2% of the stated value (or range of values), +/- 5% of the stated value (or range of values), +/-10% of the stated value (or range of values), etc. Any numerical values given herein should also be understood to include about or approximately that value, unless the context indicates otherwise. For example, if the value "10" is disclosed, then "about 10" is also disclosed. Any numerical range recited herein is intended to include all sub-ranges subsumed therein. It is also understood that when a value is disclosed that "less than or equal to" the value, "greater than or equal to the value" and possible ranges between values are also disclosed, as appropriately understood by the skilled artisan. For example, if the value "X" is disclosed the "less than or equal to X" as well as "greater than or equal to X" (e.g., where X is a numerical value) is also disclosed. It is also understood that the throughout the application, data is provided in a number of different formats, and that this data represents endpoints and starting points, and ranges for any combination of the data points. For example, if a particular data point "10" and a particular data point "15" are disclosed, it is understood that greater than, greater than or equal to, less than, less than or equal to, and equal to 10 and 15 are considered disclosed as well as between 10 and 15. It is also understood that each unit between two particular units are also disclosed. For example, if 10 and 15 are disclosed, then 11, 12, 13, and 14 are also disclosed.

Although various illustrative embodiments are described above, any of a number of changes may be made to various embodiments without departing from the scope of the invention as described by the claims. For example, the order in which various described method steps are performed may often be changed in alternative embodiments, and in other alternative embodiments one or more method steps may be skipped altogether. Optional features of various device and system embodiments may be included in some embodiments and not in others. Therefore, the foregoing description is provided primarily for exemplary purposes and should not be interpreted to limit the scope of the invention as it is set forth in the claims.

The examples and illustrations included herein show, by way of illustration and not of limitation, specific embodiments in which the subject matter may be practiced. As mentioned, other embodiments may be utilized and derived there from, such that structural and logical substitutions and changes may be made without departing from the scope of this disclosure. Such embodiments of the inventive subject matter may be referred to herein individually or collectively by the term "invention" merely for convenience and without intending to voluntarily limit the scope of this application to any single invention or inventive concept, if more than one is, in fact, disclosed. Thus, although specific embodiments have been illustrated and described herein, any arrangement calculated to achieve the same purpose may be substituted for the specific embodiments shown. This disclosure is intended to cover any and all adaptations or variations of various embodiments. Combinations of the above embodiments, and other embodiments not specifically described herein, will be apparent to those of skill in the art upon reviewing the above description.

## Claims

1. A system (1000) for applying energy to a target tissue or cell, the system comprising:
a first output (1009) configured to couple to a first one or more electrode(s) (1018);
a second output (1010) configured to couple to a second one or more electrode(s) (2019);
a third output (1008) configured to couple to a targeting electrode (1017);
a pulse generator (1003) configured to generate a first train of sub-microsecond unipolar pulses and a second train of sub-microsecond unipolar pulses, wherein the first and the second trains of sub-microsecond unipolar pulses are 180 degrees out of phase; and
a controller (1005) configured to apply the first train of sub-microsecond unipolar pulses to the first output concurrently with the second train of sub-microsecond unipolar pulses to the second output, and to couple the third output to the targeting electrode.

2. The system of claim 2, further comprising the first one or more electrode(s) coupled to the pulse generator through the first output, the second one or more electrode(s) coupled to the pulse generator through the second output, and the targeting electrode coupled to the pulse generator through the third output.

3. The system of claims 1 or 2, wherein the first one or more electrode(s) and the second one or more electrode(s) are part of an applicator (1021).

4. The system of any of claims 1-3, wherein at least one of the first one or more electrode(s), the second one or more electrode(s) and the targeting electrode comprises a needle electrode.

5. The system of any of claims 1-3, wherein at least one of the first one or more electrode(s), the second one or more electrode(s) and the targeting electrode comprises a non-penetrating electrode.

6. The system of any of claims 1-5, wherein the controller (1005) is integrated with the pulse generator (1003).

7. The system of any of claims 1-6, wherein the controller (1005) is configured to adjust an amplitude or an electric field strength of the first train of sub-microsecond unipolar pulses and the second train of the sub-microsecond unipolar pulses, optionally wherein the controller is further configured to control a depth and/or a direction of an electric field at the targeting electrode.

8. The system of any of claims 1-6, wherein the controller (1005) is configured to adjust an electric field strength of the first train of sub-microsecond unipolar pulses and the second train of sub-microsecond unipolar pulses such that an electric field strength of a summed unipolar pulse at the targeting electrode is between 0.01 kV/cm and 10 kV/cm.

9. The system of any of claims 1-8, wherein the controller (1005) is configured to adjust a burst duration of the first train of sub-microsecond unipolar pulses and the second train or sub-microsecond unipolar pulses, optionally wherein the first train of sub-microsecond unipolar pulses and the second train or sub-microsecond unipolar pulses comprises rectangular pulses.

10. The system of claim 9, wherein the controller (1005) is configured to adjust the burst duration of the first train of sub-microsecond unipolar pulses and the second train of sub-microsecond unipolar pulses so that a pulse width of a summed unipolar pulse at the targeting electrode is between 1 microsecond and 100 milliseconds.

11. The system of any of claims 1-10, wherein the first one or more electrode(s), the second one or more electrode(s) and the targeting electrode are arranged to define a triangle or a pyramid.

12. The system of any of claims 1-11, wherein the first one or more electrodes comprises a first electrode and a fourth electrode, the second one or more electrodes comprises the second electrode and the fifth electrode, the system further comprising a fourth output coupled to the fourth electrode and a fifth output coupled to the fifth electrode, wherein the controller is configured to apply the first train of sub-microsecond unipolar pulses to both the first output and the fourth output and to apply the second train of sub-microsecond unipolar pulses to both the second output and the fifth output.

13. The system of claim 12,wherein the first electrode, the second electrode, the fourth electrode and the fifth electrode are part of an applicator (1021).

14. The system of claims 12 or 13, wherein the first electrode, the second electrode, the fourth electrode, and the fifth electrode are arranged in a plane.

15. The system of claims 12 or 13, wherein the targeting electrode, the first electrode, the second electrode, the fourth electrode and the fifth electrode define a pyramid with the targeting electrode at an apex of the pyramid.

16. The system of any of claims 1-15, wherein the first and the second trains of sub-microsecond unipolar pulses sum at the target tissue or cell to form a unipolar pulse having a pulse width that is equivalent to a burst duration of the first train and the second train.

17. The system of any of claims 1-16, wherein the first train of sub-microsecond unipolar pulses is applied from both the first electrode and a third electrode and the second train of sub-microsecond unipolar pulses is applied from both the second electrode and a fourth electrode.

## Patentansprüche

1. System (1000) zum Aufbringen von Energie auf ein Zielgewebe oder eine Zielzelle, das System umfassend:
einen ersten Ausgang (1009), der konfiguriert ist, um mit einer oder mehreren ersten Elektroden (1018) gekoppelt zu werden;
einen zweiten Ausgang (1010), der konfiguriert ist, um mit einer oder mehreren zweiten Elektroden (2019) gekoppelt zu werden;
einen dritten Ausgang (1008), der konfiguriert ist, um mit einer Zielausrichtungselektrode (1017) gekoppelt zu werden;
einen Impulsgenerator (1003), der konfiguriert ist, um eine erste Folge von unipolaren Submikrosekunden-Impulsen und eine zweite Folge von unipolaren Submikrosekunden-Impulsen zu erzeugen, wobei die erste und die zweite Folge von unipolaren Submikrosekunden-Impulsen um 180 Grad phasenverschoben sind; und
eine Steuerung (1005), die konfiguriert ist, um, die erste Folge von unipolaren Submikrosekunden-Impulsen an den ersten Ausgang gleichzeitig mit der zweiten Folge von unipolaren Submikrosekunden-Impulsen anzulegen und den dritten Ausgang mit der Zielausrichtungselektrode zu koppeln.

2. System nach Anspruch 2, ferner umfassend die eine oder die mehreren ersten Elektroden, die mit dem Impulsgenerator über den ersten Ausgang gekoppelt sind, die eine oder die mehreren zweiten Elektroden, die mit dem Impulsgenerator über den zweiten Ausgang gekoppelt sind, und die Zielausrichtungselektrode, die mit dem Impulsgenerator über den dritten Ausgang gekoppelt ist.

3. System nach Anspruch 1 oder 2, wobei die eine oder die mehreren ersten Elektroden und die eine oder die mehreren zweiten Elektroden Teil eines Applikators (1021) sind.

4. System nach einem der Ansprüche 1 bis 3, wobei mindestens eine oder mehrere erste Elektroden, die eine oder die mehreren zweiten Elektroden und die Zielausrichtungselektrode eine Nadelelektrode umfassen.

5. System nach einem der Ansprüche 1 bis 3, wobei mindestens eine oder mehrere der ersten Elektroden, die eine oder die mehreren zweiten Elektroden und die Zielausrichtungselektrode eine nicht eindringende Elektrode umfassen.

6. System nach einem der Ansprüche 1 bis 5, wobei die Steuerung (1005) in den Impulsgenerator (1003) integriert ist.

7. System nach einem der Ansprüche 1 bis 6, wobei die Steuerung (1005) konfiguriert ist, um eine Amplitude oder eine elektrische Feldstärke der ersten Folge von unipolaren Submikrosekunden-Impulsen und der zweiten Folge von unipolaren Submikrosekunden-Impulsen einzustellen, optional wobei die Steuerung ferner konfiguriert ist, um eine Tiefe und/oder eine Richtung eines elektrischen Felds an der Zielausrichtungselektrode zu steuern.

8. System nach einem der Ansprüche 1 bis 6, wobei die Steuerung (1005) konfiguriert ist, um eine elektrische Feldstärke der ersten Folge von unipolaren Submikrosekunden-Impulsen und der zweiten Folge von unipolaren Submikrosekunden-Impulsen einzustellen, derart, dass eine elektrische Feldstärke eines summierten unipolaren Impulses an der Zielausrichtungselektrode zwischen 0,01 kV/cm und 10 kV/cm liegt.

9. System nach einem der Ansprüche 1 bis 8, wobei die Steuerung (1005) konfiguriert ist, um eine Impulsfolgedauer der ersten Folge von unipolaren Submikrosekunden-Impulsen und der zweiten Folge oder unipolaren Submikrosekunden-Impulsen einzustellen, optional wobei die erste Folge von unipolaren Submikrosekunden-Impulsen und die zweite Folge von unipolaren Submikrosekunden-Impulsen Rechteckimpulse umfassen.

10. System nach Anspruch 9, wobei die Steuerung (1005) konfiguriert ist, um die Impulsfolgedauer der ersten Folge von unipolaren Submikrosekunden-Impulsen und der zweiten Folge von unipolaren Submikrosekunden-Impulsen einzustellen, derart, dass eine Impulsbreite eines summierten unipolaren Impulses an der Zielausrichtungselektrode zwischen 1 Mikrosekunde und 100 Millisekunden liegt.

11. System nach einem der Ansprüche 1 bis 10, wobei die eine oder die mehreren ersten Elektroden, die eine oder die mehreren zweiten Elektroden und die Zielausrichtungselektrode angeordnet sind, um ein Dreieck oder eine Pyramide zu definieren.

12. System nach einem der Ansprüche 1 bis 11, wobei die eine oder die mehreren ersten Elektroden eine erste Elektrode und eine vierte Elektrode umfassen, die eine oder die mehreren zweiten Elektroden die zweite Elektrode und die fünfte Elektrode umfassen, das System ferner umfassend einen vierten Ausgang, der mit der vierten Elektrode gekoppelt ist, und einen fünften Ausgang, der mit der fünften Elektrode gekoppelt ist, wobei die Steuerung konfiguriert ist, um die erste Folge von unipolaren Submikrosekunden-Impulsen sowohl an den ersten Ausgang als auch an den vierten Ausgang anzulegen und die zweite Folge von unipolaren Submikrosekunden-Impulsen sowohl an den zweiten Ausgang als auch an den fünften Ausgang anzulegen.

13. System nach Anspruch 12, wobei die erste Elektrode, die zweite Elektrode, die vierte Elektrode und die fünfte Elektrode Teil eines Applikators (1021) sind.

14. System nach Anspruch 12 oder 13, wobei die erste Elektrode, die zweite Elektrode, die vierte Elektrode und die fünfte Elektrode in einer Ebene angeordnet sind.

15. System nach Anspruch 12 oder 13, wobei die Zielausrichtungselektrode, die erste Elektrode, die zweite Elektrode, die vierte Elektrode und die fünfte Elektrode eine Pyramide definieren, wobei die Zielausrichtungselektrode an der Spitze der Pyramide ist.

16. System nach einem der Ansprüche 1 bis 15, wobei sich die erste und die zweite Folge von unipolaren Submikrosekunden-Impulsen an dem Zielgewebe oder der Zielzelle summieren, um einen unipolaren Impuls auszubilden, der eine Breite aufweist, die gleich einer Impulsfolgedauer der ersten Folge und der zweiten Folge ist.

17. System nach einem der Ansprüche 1 bis 16, wobei die erste Folge von unipolaren Submikrosekunden-Impulsen sowohl von der ersten Elektrode als auch von einer dritten Elektrode angelegt wird und die zweite Folge von unipolaren Submikrosekunden-Impulsen sowohl von der zweiten Elektrode als auch von einer vierten Elektrode angelegt wird.

## Revendications

1. Système (1000) permettant d'appliquer de l'énergie à un tissu ou une cellule cible, le système comprenant :
une première sortie (1009) configurée pour se coupler à une première ou plusieurs électrode(s) (1018) ;
une deuxième sortie (1010) configurée pour se coupler à une deuxième ou plusieurs électrode(s) (2019) ;
une troisième sortie (1008) configurée pour se coupler à une électrode de ciblage (1017) ;
un générateur d'impulsions (1003) configuré pour générer un premier train d'impulsions unipolaires sub-microsecondes et un second train d'impulsions unipolaires sub-microsecondes, dans lequel le premier et le second trains d'impulsions unipolaires sub-microsecondes sont déphasés de 180 degrés ; et
un dispositif de commande (1005) configuré pour appliquer le premier train d'impulsions unipolaires sub-microsecondes à la première sortie simultanément avec le second train d'impulsions unipolaires sub-microsecondes à la deuxième sortie, et pour coupler la troisième sortie à l'électrode de ciblage.

2. Système selon la revendication 2, comprenant en outre la première ou plusieurs électrode(s) couplée(s) au générateur d'impulsions par le biais de la première sortie, la deuxième ou plusieurs électrode(s) couplée(s) au générateur d'impulsions par le biais de la deuxième sortie, et l'électrode de ciblage couplée au générateur d'impulsions par le biais de la troisième sortie.

3. Système selon les revendications 1 ou 2, dans lequel la première ou plusieurs électrode(s) et la deuxième ou plusieurs électrode(s) font partie d'un applicateur (1021).

4. Système selon l'une quelconque des revendications 1 à 3, dans lequel au moins l'une parmi la première ou plusieurs électrode(s), la deuxième ou plusieurs électrode(s) et l'électrode de ciblage comprend une électrode aiguille.

5. Système selon l'une quelconque des revendications 1 à 3, dans lequel au moins l'une parmi la première ou plusieurs électrode(s), la deuxième ou plusieurs électrode(s) et l'électrode de ciblage comprend une électrode non pénétrante.

6. Système selon l'une quelconque des revendications 1 à 5, dans lequel le dispositif de commande (1005) est intégré au générateur d'impulsions (1003).

7. Système selon l'une quelconque des revendications 1 à 6, dans lequel le dispositif de commande (1005) est configuré pour ajuster une amplitude ou une intensité de champ électrique du premier train d'impulsions unipolaires sub-microsecondes et du second train d'impulsions unipolaires sub-microsecondes, éventuellement dans lequel le dispositif de commande est en outre configuré pour commander une profondeur et/ou une direction d'un champ électrique au niveau de l'électrode de ciblage.

8. Système selon l'une quelconque des revendications 1 à 6, dans lequel le dispositif de commande (1005) est configuré pour ajuster une intensité de champ électrique du premier train d'impulsions unipolaires sub-microsecondes et du second train d'impulsions unipolaires sub-microsecondes de telle sorte qu'une intensité de champ électrique d'une impulsion unipolaire sommée au niveau de l'électrode de ciblage est entre 0,01 kV/cm et 10 kV/cm.

9. Système selon l'une quelconque des revendications 1 à 8, dans lequel le dispositif de commande (1005) est configuré pour ajuster une durée de rafale du premier train d'impulsions unipolaires sub-microsecondes et du second train ou des impulsions unipolaires sub-microsecondes, éventuellement dans lequel le premier train d'impulsions unipolaires sub-microsecondes et le second train ou les impulsions unipolaires sub-microsecondes comprennent des impulsions rectangulaires.

10. Système selon la revendication 9, dans lequel le dispositif de commande (1005) est configuré pour ajuster la durée de la rafale du premier train d'impulsions unipolaires sub-microsecondes et du second train d'impulsions unipolaires sub-microsecondes de telle sorte qu'une largeur d'impulsion d'une impulsion unipolaire sommée au niveau de l'électrode de ciblage est entre 1 microseconde et 100 millisecondes.

11. Système selon l'une quelconque des revendications 1 à 10, dans lequel la première ou plusieurs électrode(s), la deuxième ou plusieurs électrode(s) et l'électrode de ciblage sont agencées pour définir un triangle ou une pyramide.

12. Système selon l'une quelconque des revendications 1 à 11, dans lequel la première ou plusieurs électrodes comprend une première électrode et une quatrième électrode, la deuxième ou plusieurs électrodes comprend la deuxième électrode et la cinquième électrode, le système comprenant en outre une quatrième sortie couplée à la quatrième électrode et une cinquième sortie couplée à la cinquième électrode, dans lequel le dispositif de commande est configuré pour appliquer le premier train d'impulsions unipolaires sub-microsecondes à la fois à la première sortie et à la quatrième sortie et pour appliquer le second train d'impulsions unipolaires sub-microsecondes à la fois à la deuxième sortie et à la cinquième sortie.

13. Système selon la revendication 12, dans lequel la première électrode, la deuxième électrode, la quatrième électrode et la cinquième électrode font partie d'un applicateur (1021).

14. Système selon les revendications 12 ou 13, dans lequel la première électrode, la deuxième électrode, la quatrième électrode, et la cinquième électrode sont agencées dans un plan.

15. Système selon les revendications 12 ou 13, dans lequel l'électrode de ciblage, la première électrode, la deuxième électrode, la quatrième électrode et la cinquième électrode définissent une pyramide avec l'électrode de ciblage au niveau d'un sommet de la pyramide.

16. Système selon l'une quelconque des revendications 1 à 15, dans lequel le premier et le second trains d'impulsions unipolaires sub-microsecondes se somment au niveau du tissu ou de la cellule cible pour former une impulsion unipolaire ayant une largeur d'impulsion qui est équivalente à une durée de rafale du premier train et du second train.

17. Système selon l'une quelconque des revendications 1 à 16, dans lequel le premier train d'impulsions unipolaires sub-microsecondes est appliqué à la fois à partir de la première électrode et d'une troisième électrode et le second train d'impulsions unipolaires sub-microsecondes est appliqué à la fois à partir de la deuxième électrode et d'une quatrième électrode.
